(19) **Europäisches Patentamt / European Patent Office / Office européen des brevets**

(11) **EP 3 455 793 B1**

(12) # EUROPEAN PATENT SPECIFICATION

(45) Date of publication and mention
of the grant of the patent:
**20.08.2025 Bulletin 2025/34**

(21) Application number: **17722887.1**

(22) Date of filing: **10.05.2017**

(51) International Patent Classification (IPC):
**G06F 18/2137** *(2023.01)* **G06F 18/20** *(2023.01)*

(52) Cooperative Patent Classification (CPC):
**G06F 18/21375; G06F 18/295;** G06V 2201/03

(86) International application number:
**PCT/IB2017/052722**

(87) International publication number:
**WO 2017/195126 (16.11.2017 Gazette 2017/46)**

(54) **A METHOD FOR DETERMINING THE TEMPORAL PROGRESSION OF A BIOLOGICAL PHENOMENON AND ASSOCIATED METHODS AND DEVICES**

VERFAHREN ZUR BESTIMMUNG DES ZEITLICHEN FORTSCHRITTS EINES BIOLOGISCHEN PHÄNOMENS UND ZUGEHÖRIGE VERFAHREN UND VORRICHTUNGEN

PROCÉDÉ DE DÉTERMINATION DE LA PROGRESSION DANS LE TEMPS D'UN PHÉNOMÈNE BIOLOGIQUE, ET PROCÉDÉS ET DISPOSITIFS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2016 PCT/IB2016/052699**

(43) Date of publication of application:
**20.03.2019 Bulletin 2019/12**

(73) Proprietors:
• **Institut National de la Santé et de la Recherche Médicale (INSERM)**
**75013 Paris (FR)**
• **Institut du Cerveau et de la Moelle Epiniere**
**75013 Paris (FR)**
• **Ecole Polytechnique**
**91120 Palaiseau (FR)**
• **INRIA - Institut National de Recherche en Informatique et en Automatique**
**78150 Le Chesnay (FR)**
• **Centre National de la Recherche Scientifique**
**75016 Paris (FR)**
• **SORBONNE UNIVERSITE**
**75006 Paris (FR)**
• **Assistance Publique - Hôpitaux de Paris**
**75012 Paris (FR)**

(72) Inventors:
• **DURRLEMAN, Stanley**
**75252 Paris Cedex 05 (FR)**
• **SCHIRATTI, Jean-Baptiste**
**91128 Palaiseau Cedex (FR)**
• **ALLASSONNIERE, Stéphanie**
**75006 Paris (FR)**
• **COLLIOT, Olivier**
**75252 Paris Cedex 05 (FR)**

(74) Representative: **Lavoix**
**2, place d'Estienne d'Orves**
**75441 Paris Cedex 09 (FR)**

(56) References cited:
**US-A1- 2004 242 972**

• **J.-B SCHIRATTI ET AL: "Mixed-effects model for the spatiotemporal analysis of longitudinal manifold-valued data", MATHEMATICAL FOUNDATIONS OF COMPUTATIONAL ANATOMY, 9 October 2015 (2015-10-09), pages 48 - 59, XP055333442**

• BILGEL MURAT ET AL: "A multivariate nonlinear mixed effects model for longitudinal image analysis: Application to amyloid imaging", NEUROIMAGE, ELSEVIER, AMSTERDAM, NL, vol. 134, 16 April 2016 (2016-04-16), pages 658 - 670, XP029608533, ISSN: 1053-8119, DOI: 10.1016/J.NEUROIMAGE.2016.04.001

**Description**

TECHNICAL FIELD OF THE INVENTION

The present invention concerns a method for determining the temporal progression of a biological phenomenon. The present invention also relates to an associated method for predicting, an associated method for diagnosing, an associated method for identifying a therapeutic target, an associated method for identifying a biomarker and an associated method for screening. The present invention also concerns a computer program product and a computer readable medium adapted to carry out one of these methods.

BACKGROUND OF THE INVENTION

Age-related brain diseases, such as Parkinson's or Alzheimer's disease are complex diseases, which have multiple effects on the metabolism, structure and function of the brain. Models of disease progression showing the sequence and timing of these effects during the course of the disease remain largely hypothetical. Large multimodal databases have been collected in the recent years in the hope to give experimental evidence of the patterns of disease progression based on the estimation of data-driven models. These databases are longitudinal, in the sense that they contain repeated measurements of several subjects at multiple time-points which do not necessarily correspond across subjects. As a matter of fact, learning models of disease progression from such databases raises great methodological challenges.

BILGEL MURAT et al. "A multivariate nonlinear mixed effects model for longitudinal image analysis : Application to amyloid imaging" NEUROIMAGE, ELSEVIER, AMSTERDAM, NL, vol. 134, 18 April 2016 (2016-04-16), pages 658-670, ISSN: 1053-8119, DOI: 10.1016/J.NEUROIMAGE.2016.04.001 or US 2004/242972 A1 are examples of such models of disease progression.

The main difficulty lies in the fact that the age of a given individual gives no information about the stage of disease progression of this individual. The first clinical symptoms of Alzheimer's disease may appear at forty years for one patient and eighty years for another. The duration of the disease also may vary across patients from few years to decades. Moreover, the onset of the disease does not correspond with the onset of the symptoms: according to recent studies, symptoms are likely to be preceded by a silent phase of the disease, for which little is known. As a consequence, statistical models based on the regression of measurements with age are inadequate to model disease progression.

In "Mixed-effects model for the spatiotemporal analysis of longitudinal manifold-valued data", MATHEMATICAL FOUNDATIONS OF COMPUTATIONAL ANATOMY, 9 October 2015 (2016-10-09), pages 48-59, Schiratti et al. disclose a Riemannian manifold for modelling temporal progression of neurodegenerative diseases.

SUMMARY OF THE INVENTION

The invention aims at determining the temporal progression of a biological phenomenon, notably a neurodegenerative disease, based on data taken from a subject.

To this end, the invention concerns a method for determining the temporal progression of a biological phenomenon which may affect a studied subject, the method being as defined in claim 1.

The present invention enables to determine the temporal progression of a biological phenomenon based on data taken from a subject.

The data taken from the subject are biomarkers for the studied subject, the biomarkers being relative to the progression of the biological phenomenon.

There is little constraint on the data taken from the subject which enables to carry out the method with data taken from the subject different from the data used to build the numerical model. The only constraint is that the data taken from the subject and the data used to build the numerical model be relative to the same biomarker(s).

The converting step ensures such freedom.

In addition, the numerical model enables to obtain interesting result by proposing a generic statistical framework for the definition and estimation of mixed-effects models for longitudinal manifold-valued data. Using the tools of geometry allows us to derive a method that makes little assumptions about the data and problem to deal with. Modeling choices boil down to the definition of the metric on the manifold. This geometrical modeling also allows us to introduce the concept of parallel curves on a manifold, which is a key to decompose differences seen in the data in a unique manner into a spatial and a temporal component. Because of the non-linearity of the model, the estimation of the parameters shall be based on an adequate maximization of the observed likelihood. To address this issue, a stochastic version of the Expectation-Maximization algorithm is used.

According to further aspects of the invention which are advantageous but not compulsory, the method for determining the temporal progression of a biological phenomenon might incorporate one or several of the following features, taken in any technically admissible combination:

- the biological phenomenon is a biological phenomenon whose temporal progression extends over more than three years.
- the biological phenomenon is a neurodegenerative disease.
- at the step of providing first data, the first data are neuropsychological biomarkers.
- the studied subject is an animal, preferably a mammal, more preferably a human being.
- at the step of providing the numerical model, the stochastic approximation in an expectation-maximization technique is a Monte-Carlo Markov Chain Stochastic Approximation Expectation-Maximization technique.
- at the step of providing the numerical model, the number of subjects is superior or equal to 100.
- in the numerical model, the data relative to the dispersion of the progression trajectory for the biological phenomenon among a plurality of subjects are provided as standard deviations of time for a plurality of values of biomarkers.

The invention also relates to a method for predicting that a subject is at risk of suffering from a disease, the method for predicting at least comprising the step of carrying out the steps of the method for determining the temporal progression of a biological phenomenon which may affect a studied subject as previously described, the biological phenomenon being the disease, to obtain a first temporal progression, and the step of predicting that the subject is at risk of suffering from the disease based on the first temporal progression.

The invention also relates to a method for diagnosing a disease, the method for diagnosing at least comprising the step of carrying out the steps of the method for determining the temporal progression of a biological phenomenon which may affect a studied subject as previously described, the biological phenomenon being the disease, to obtain a first temporal progression, and the step of diagnosing the disease based on the first temporal progression.

The invention also relates to a method for identifying a biomarker the biomarker being a diagnostic biomarker of a pathology, a susceptibility biomarker of a pathology, a prognostic biomarker of a pathology or a predictive biomarker in response to the treatment of a pathology, the method comprising the steps of carrying out the method for determining the temporal progression of a biological phenomenon which may affect a studied subject as previously described, the first data being data relative to a subject suffering from the pathology, to obtain a first temporal progression, carrying out the method for determining the temporal progression of a biological phenomenon which may affect a studied subject as previously described, the first data being data relative to a subject not suffering from the pathology, to obtain a second temporal progression,

selecting a biomarker based on the comparison of the first and second temporal progressions.

The invention also relates to a computer program product comprising instructions for carrying out the steps of a method as previously described, when said computer program product is executed on a suitable computer device. The invention also relates to a computer readable medium having encoded thereon a computer program product as previously described.

BRIEF DESCRIPTION OF THE DRAWINGS

The invention will be better understood on the basis of the following description which is given in correspondence with the annexed figures. In the annexed figures:

- Figure 1 shows schematically a system and a computer program product whose interaction enables to carry out a method for determining the temporal progression of a biological phenomenon;
- Figure 2 shows a flowchart of an example of carrying out a method for determining the temporal progression of a biological phenomenon;
- Figure 3 shows schematically an example of longitudinal manifold-valued data;
- Figure 4 shows schematically an example of numerical model in a Riemann manifold;
- Figures 5 to 7 shows schematically calculation in the Riemann manifold so as to obtain another example of numerical model, and
- Figures 8 to 16 show schematically experimental results obtained by using the numerical model of Figures 5 to 7.

DETAILED DESCRIPTION OF SOME EMBODIMENTS

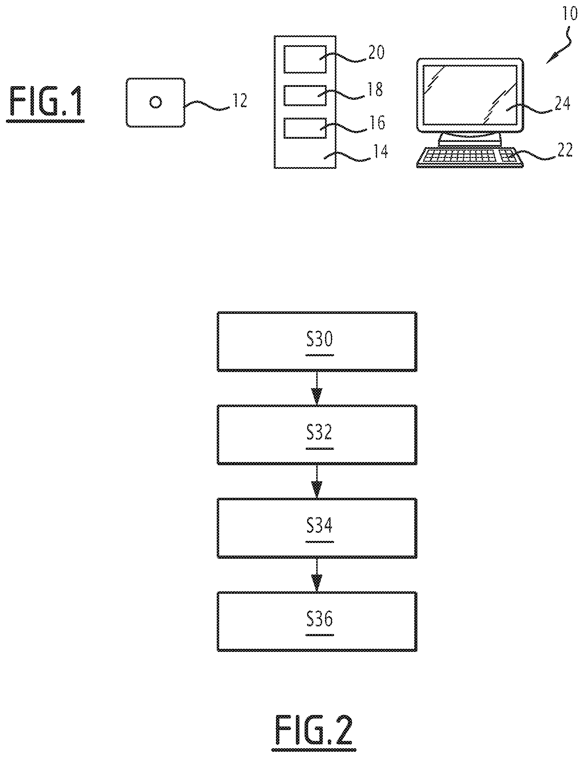

A system 10 and a computer program product 12 are represented in Figure 1. The interaction between the computer program product 12 and the system 10 enables to carry out a method for determining the temporal progression of a biological phenomenon.

System 10 is a computer. In the present case, system 10 is a laptop.

More generally, system 10 is a computer or computing system, or similar electronic computing device adapted to manipulate and/or transform data represented as physical, such as electronic, quantities within the computing system's registers and/or memories into other data similarly represented as physical quantities within the computing system's memories, registers or other such information storage, transmission or display devices.

System 10 comprises a processor 14, a keyboard 22 and a display unit 24.

The processor 14 comprises a data-processing unit 16, memories 18 and a reader 20. The reader 20 is adapted to read a computer readable medium.

The computer program product 12 comprises a computer readable medium.

The computer readable medium is a medium that can be read by the reader of the processor. The computer readable medium is a medium suitable for storing electronic instructions, and capable of being coupled to a computer system bus.

Such computer readable storage medium is, for instance, a disk, a floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

A computer program is stored in the computer readable storage medium. The computer program comprises one or more stored sequence of program instructions.

The computer program is loadable into the data-processing unit 16 and adapted to cause execution of the method for determining the temporal progression of a biological phenomenon when the computer program is run by the data-processing unit 16.

Operation of the system 10 is now described in reference to an example of carrying out of a method for determining the temporal progression of a biological phenomenon which may affect a studied subject.

The studied subject is an animal.

Preferably, the studied subject is a mammal.

More preferably, the studied subject is a human being.

For the sake of exemplification, it is assumed, in the remainder of the specification, that the studied subject is a human being.

In the most generic definition, the biological phenomenon is a biological phenomenon for which a temporal progression can be defined.

In the present claimed invention, the biological phenomenon is a disease or the ageing.

According to a specific embodiment, the biological phenomenon is a biological phenomenon whose temporal progression extends over more than three years.

According to a more specific embodiment, the biological phenomenon is a biological phenomenon whose temporal progression extends over more than ten years.

In addition, at least one of the onset of the biological phenomenon or the duration of the biological phenomenon varies from a subject to another.

A typical example for such biological phenomenon is a neurodegenerative disease.

Neurodegenerative disease designates a set of disease which primarily affects the neurons in the human brain.

Alzheimer's disease, Parkinson's disease, prion disease, motor neurone disease, Huntington's disease, spinocerebellar ataxia and spinal muscular atrophy are examples of neurodegenerative diseases.

For the sake of exemplification, it is assumed, in the remainder of the specification, that the biological phenomenon is Alzheimer's disease.

The method for determining comprises four steps: a step S30 of providing first data, a step S32 of providing a numerical model, a step S34 of converting and a step S36 of using.

At step S30, first data are provided.

The first data are biomarkers for the studied subject.

The biomarkers are relative to the progression of the biological phenomenon.

For instance, the first data are data collected via cognitive tests devoted to detect the Alzheimer's disease.

According to another embodiment, the first data are data obtained by medical imaging.

According to still another embodiment, the first data are a combination of data collected by different ways. As an example, the first data are data collected via cognitive tests and data by medical imaging.

Preferably, the first data are data collected at several time points for the same subject.

In the specific described example, the first data are neuropsychological biomarkers, for instance assessing cognitive or motor functions such as memory or praxis.

At step S32, a numerical model labeled NM is provided.

The numerical model NM is a function in a Riemann manifold named RM.

The numerical model NM associates to values of biomarkers a temporal progression trajectory for the biological phenomenon and data relative to the dispersion of the progression trajectory for the biological phenomenon among a plurality of subjects.

According to a preferred embodiment, the data relative to the dispersion of the progression trajectory for the biological phenomenon among a plurality of subjects are provided as standard deviations of time for a plurality of values of biomarkers.

The numerical model NM is therefore construable as a statistical model.

The numerical model NM is obtained by using a stochastic approximation in an expectation-maximization technique on data relative to biomarkers taken at different time points for a plurality of subjects.

This means that the numerical model NM concerns longitudinal data.

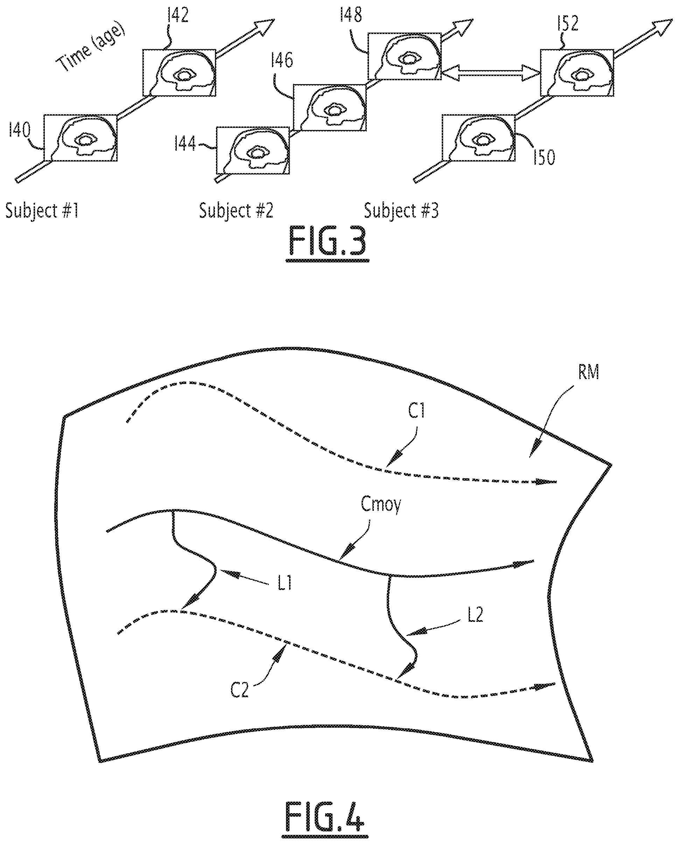

Figure 3 illustrates a specific example of longitudinal data.

For each subject, several images of their brain are taken at different instants.

More precisely, for subject 1, two images I40 and I42 are provided.

For subject 2, three images I44, I46 and I48 are provided.

For subject 3, two images I50 and I52 are provided.

Except for images I48 and I52 (as schematically illustrated by the arrow on Figure 3), the images are taken at different time points.

The case of Figure 3 is purely illustrative, the longitudinal data being usually more numerous.

Indeed, preferably, the number of subjects is superior or equal to 100.

In other words, the data consists in repeated multivariate measurements of $p$ individuals. For a given individual, the measurements are obtained at time points $t_{i,1} < ... < t_{i,ni}$. The $j$-th measurement of the $i$-th individual is denoted by $\mathbf{y}_{i,j}$. In the remainder of the specification, it is assumed that each observation $\mathbf{y}_{i,j}$ is a point on a $N$-dimensional Riemannian manifold $\mathbb{M}$ embedded in $\mathbb{R}^N$ and equipped with a Riemannian metric $g^{\mathbb{M}}$.

The generic spatiotemporal model belongs to a class of statistical models for which maximum likelihood estimates cannot be obtained in closed form. This issue is addressed by using a stochastic approximation in a expectation-maximization technique.

Preferably, the stochastic approximation in an expectation-maximization technique is a Monte-Carlo Markov Chain Stochastic Approximation Expectation-Maximization technique.

An example of the numerical model NM provided at the step S32 is schematically represented on Figure 4.

The numerical model NM can be provided by providing three curves which are $C_{moy}$, $C_1$ and $C_2$. The three curves correspond to three kinds of temporal progression for the Alzheimer's disease. These curves may represent the average disease progression trajectory ($C_{moy}$) and the dispersion of this trajectory at plus or minus one standard deviation in the values of biomarkers for a plurality of subjects ($C_1$ and $C_2$).

Two curves L1 and L2 are represented on Figure 4. These curves indicate a temporal correspondence between the mean curve $C_{moy}$ and the second curve $C_2$.

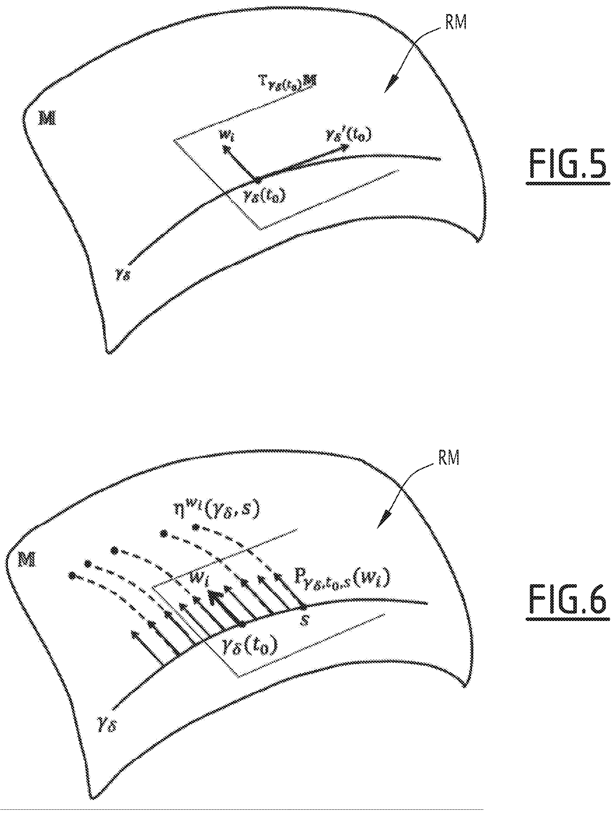

As a specific example, in relation to Figures 5 to 7, it is now illustrated how to obtain a specific numerical model NM relevant for the Alzheimer's disease.

Let ($\mathbb{M}$, $g^{\mathbb{M}}$) be a Riemannian manifold of dimension $N$ equipped with a Riemannian metric $g^{\mathbb{M}}$, which is assumed to be geodesically complete.

A Riemannian metric is geodesically complete if the geodesics of $\mathbb{M}$ are defined on $\mathbb{R}$, this notation being for the set of real numbers. It is recalled that a geodesic is a curve drawn on the manifold $\gamma : \mathbb{R} \rightarrow \mathbb{M}$, which has no acceleration.

The Riemannian metric $g^{\mathbb{M}}$ defines a unique affine connexion on $\mathbb{M}$, namely the Levi-Civita connexion, denoted by $\nabla^{\mathbb{M}}$. Let y denote a geodesic of $\mathbb{M}$ and $t_0 \in \mathbb{R}$. It is recalled that, given a tangent vector $\xi$ in $T_{\gamma(t_0)}\mathbb{M}$, the parallel transport of $\xi$ along $\gamma$, denoted by $X(s) = P_{\gamma,t0,s}(\xi)$, is a vector field along $\gamma$ which satisfies the following equations:

$$X(t_0) = \boldsymbol{\xi} \text{ and } \nabla^{\mathbb{M}} X(s) = 0$$

Let $\mathbf{p} \in \mathbb{M}$.

The Riemannian exponential in $\mathbb{M}$ at $\mathbf{p}$ is denoted by $exp_p^{\mathbb{M}}$.

For $\mathbf{v} \in T_p\mathbb{M}$, $exp_{\boldsymbol{p}}^{\mathbb{M}}(\boldsymbol{v})$ denotes the value at time 1 of the geodesic in $\mathbb{M}$ issued from $\mathbf{p}$ with initial velocity $\mathbf{v}$.

The temporal progression of a family of $N$ ($N \geq 2$) scalar biomarkers is studied. A longitudinal dataset of the form $(\mathbf{y}_{i,j}, t_{i,j})_{i,j}$ is considered. This longitudinal dataset is obtained by observing $p$ individuals at repeated time points. The vector $\mathbf{y}_{i,j}$ denotes the $j$-th observation ($1 \leq j \leq n_j$) of the $i$-th individual. The $k$-th coordinate of $\mathbf{y}_{i,j}$, denoted by $y_{i,j,k}$, corresponds to the measurement of the $k$-th biomarker, at time $t_{i,j}$.

It is assumed that each measurement $y_{i,j,k}$ belongs to a one dimensional Riemannian manifold $(M, g)$ which is geodesically complete. In this setting, the observations $\mathbf{y}_{i,j} = (y_{i,j,1},...,y_{i,j,N})$ can be considered as points in the product manifold $\mathbb{M} = M^N$. The average progression of this family of biomarkers is modeled by a geodesic trajectory on the manifold $\mathbb{M}$, which is equipped with the product metric, denoted by $g^{\mathbb{M}}$.

The numerical model NM is described for observations on a manifold which is a product of one-dimensional manifolds. This framework is particularly convenient to analyze the temporal progression of a family of biomarkers.

In order to determine relative progression of the biomarkers among themselves, the average trajectory is chosen among the parametric family of geodesics :

$$(t \rightarrow (\gamma_0(t), \gamma_0(t + \delta_1),...,\gamma_0(t + \delta_{N-1})))_\delta$$

where:

- $\delta = (0, \delta_1,...,\delta_{N-1})$ and
- $\gamma_0$ is a geodesic, of the one-dimensional manifold $M$, parametrized by a point $p_0$ in $M$, a time $t_0$ and a velocity $v_0$ in $T_{p0}M$.

This parametrization of the geodesic $\gamma_0$ is the natural parametrization such that:

$$\gamma_0(t_0) = p_0 \text{ and } \dot{\gamma}(t_0) = v_0$$

By choosing the average trajectory among this parametrized family of geodesics, it is assumed that, on average, the biomarkers follow the same trajectory but shifted in time. The delay between the progression of the different biomarkers is measured by the vector $\boldsymbol{\delta} = (0, \delta_1,...,\delta_{N-1}) \in \mathbb{R}^N$. The parameters $\delta_i$ ($1 \leq i \leq N$ - 1) measure a relative delay between two consecutive biomarkers. The parameter $t_0$ plays the role of reference time as the trajectory of the first biomarker will reach the value $p_0$ at time $t_0$ whereas the other trajectories will reach the same value $p_0$ at different points in time, shifted with respect to the time $t_0$.

The numerical model NM is a hierarchical model: data points are assumed to be sampled from subject-specific trajectories of progression. These individual trajectories are derived from the average trajectory $\gamma_\delta$. The subject-specific trajectory of the $i$-th individual is constructed by considering a non-zero tangent vector $\mathbf{w}_i$ in $T_{\gamma_\delta(t_0)}\mathbb{M}$, orthogonal to $\dot{\gamma}_\delta(t_0)$ for the inner product defined by the metric $(\langle .,.\rangle_{\gamma_\delta(t_0)} = g^{\mathbb{M}}_{\gamma_\delta(t_0)})$. This tangent vector $\mathbf{w}_i = (w_{1,i},...,w_{N,i})$ is a space shift which enables registering the individual trajectories in the space of measurements. The tangent vector $\mathbf{w}_i$ is transported along the geodesic $\gamma_\delta$ from time $t_0$ to time $s$ using parallel transport. This transported tangent vector is denoted by $P_{\gamma,t0,s}(\mathbf{w}_i)$.

At the point $\gamma_\delta(s)$, a new point in $\mathbb{M}$ is obtained by taking the Riemannian exponential of $P_{\gamma,t0,s}(\mathbf{w}_i)$. This new point is denoted by $\eta^{wi}(\gamma_\delta, s)$. As $s$ varies, this point describes the curve $s \rightarrow \eta^{wi}(\gamma_\delta, s)$, which is considered as a "parallel" to the curve $\gamma_\delta$ (see Figure 5). The orthogonality condition on the tangent vectors $\mathbf{w}_i$ is an important hypothesis which ensures that a point $\eta^{wi}(\gamma_\delta, s)$ on a parallel moves at the same pace on this parallel than on the average trajectory. This hypothesis ensures the uniqueness of the decomposition between spatial and temporal components.

The trajectory $\gamma_i$ of the $i$-th individual is obtained by reparametrizing the parallel $\eta^{wi}(\gamma_\delta,.) : \gamma_i(s) = \eta^{wi}(\gamma_\delta, \psi_i(s))$, where the mapping $\psi_i(s) = \alpha_i(s - t_0 - \tau_i) + t_0$ is a subject-specific affine reparametrization which allows registering in time the different individual trajectories of progression. Such affine reparametrization corresponds to a time-warp wherein the tangent vector $\mathbf{w}_i$ can be considered, in the light of the univariate model, as a random effect associated to the point $p_0$.

The parameter $\alpha_i$ is an acceleration factor which encodes whether the $i$-th individual is progressing faster or slower than the average, $\tau_i$ is a time-shift which characterizes the advance or delay of the $ith$ individual with respect to the average and $\mathbf{w}_i$ is a space-shift which encodes the variability in the measurements across individuals at a given stage, once the paces at which individual trajectories are followed are normalized. Each of these parameters are assumed to be random, non observed and variables.

Because $\mathbb{M}$ is equipped with the product metric, the parallel transport of the tangent vector $\mathbf{w}_i \in T_{\gamma_\delta(t_0)}\mathbb{M}$ is a $N$-dimensional vector whose $k$-th ($1 \leq k \leq N$) component is equal to the parallel transport of the tangent vector $w_{k,i} \in T_{\gamma_0(.+\delta_{k-1})}\mathbb{M}$ along the curve $s \rightarrow \gamma_0(s + \delta_{k-1})$ in the one-dimensional manifold $M$. It follows that:

$$P_{\gamma,t_0,s}(\mathbf{w}_i) = \left(\frac{w_{1,i}}{\dot{\gamma}_0(t_0)}\dot{\gamma}_0(s), ..., \frac{w_{N,i}}{\dot{\gamma}_0(t + \delta_{N-1})}\dot{\gamma}_0(s + \delta_{N-1})\right)$$

Taking the Riemanniann exponential, in $\mathbb{M}$, of the tangent vector $P_{y,t0,s}(w_i)$ boils down to taking the Riemannian exponential, in M, of each component of the vector. If $exp^{\mathbb{M}}$ denotes the Riemannian exponential map in M, the $k$-th ($1 \le k \le N$) component of $\eta^{wi}(\gamma_\delta, s)$ is given by :

$$exp^{\mathbb{M}}_{\gamma_0(s+\delta_{k-1})} \left[ \frac{w_{k,i}}{\dot{\gamma}_0(t_0+\delta_{k-1})} \dot{\gamma}_0(s + \delta_{N-1}) \right] = \gamma_0 \left( s + \delta_{k-1} + \frac{w_{k,i}}{\dot{\gamma}_0(t_0+\delta_{k-1})} \right)$$

For the longitudinal dataset $(y_{i,j}, t_{i,j})$ ($1 \le i \le p$, $1 \le j \le n_i$), the numerical model NM writes:

$$\mathbf{y}_{i,j} = \gamma_i(t_{i,j}) + \varepsilon_{i,j}$$

In particular, for the k-th biomarker, this numerical model NM writes :

$$y_{i,j,k} = \gamma_0 \left( \frac{w_{k,i}}{\dot{\gamma}_0(t_0 + \delta_{k-1})} + t_0 + \alpha_i(s - t_0 - \tau_i) + \delta_{k-1} \right) + \epsilon_{i,j,k}$$

Wherein:

- $\alpha_i = exp(\eta_i)$ with $\eta_i$ is assumed to follow a probability distribution $\mathcal{N}\left(0, \sigma_\eta^2\right)$, $\mathcal{N}$ being the Gaussian distribution with zero mean and variance $\sigma_\eta^2$,

- $\tau_i$ is assumed to follow a probability distribution according to $\mathcal{N}(0, \sigma_\tau^2)$, $\sigma_\tau^2$ being the variance of the Gaussian distribution,

- $\varepsilon_{i,j}$ is assumed to follow a probability distribution according to $\mathcal{N}(0, \sigma^2 \cdot I_n)$, $\sigma^2$ being the variance of the Gaussian distribution and $I_n$ the identity matrix of order n, and

- the tangent vectors $w_i$ are assumed to be a linear combination of $N_s < N$ statistically independent components. This writes $\mathbf{w}_i = A.\mathbf{s}_i$ where A is a $N \times N_s$ matrix of rank $N_s$ whose columns are vectors in $T_{\gamma_\delta(t_0)}\mathbb{M}$ and $\mathbf{s}_i$ is a vector of $N_s$ independent sources following a Laplace distribution with parameter 1/2.

As a consequence, the fixed effects of the model are the parameters of the average geodesic: the point $\mathbf{p_0}$ on the manifold, the time-point $t_0$ and the velocity $\mathbf{v_0}$. The random effects are the acceleration factors $\alpha_i$, time-shifts $\tau_i$ and space-shifts $\mathbf{w}_i$. The random effects $\mathbf{z} = (\eta_i, \tau_i, s_{j,i})$ ($1 \le i \le p$ and $1 \le j \le N_s$) are considered as hidden variables. With the observed data $\mathbf{y} = (y_{i,j,k})_{i,j,k}$, $(\mathbf{y}, \mathbf{z})$ form the complete data of the model. In this context, the Expectation-Maximization (EM) algorithm is very efficient to compute the maximum likelihood estimate of the parameters of the model, denoted θ.

In other words, it appears that the numerical model NM depends from the vector parameter θ which writes:

$$\boldsymbol{\theta} = \left( p_0, t_0, v_0, \boldsymbol{\delta}, \sigma_\eta, \sigma_\tau, \sigma, vec(A) \right)$$

where vec(A) stands for the entries of the matrix A concatenated in a raw vector.

In addition, the random effects of the model are described by $(\alpha_i, \tau_i, \mathbf{w}_i)$ ($1 \le i \le p$).

A stochastic version of the Expectation-Maximization (EM) algorithm is used to estimate the vector parameter θ of the numerical model NM. Because of the nonlinearity of the model, the E step of the EM algorithm is intractable. A stochastic version of the EM algorithm, namely the Monte-Carlo Markov Chain Stochastic Approximation Expectation-Maximization (MCMC-SAEM) algorithm is used.

In order to ensure the theoretical convergence of the MCMC SAEM algorithm, the parameters of the model are considered as realizations of independents Gaussian random variables, which is equivalent to ensure that the model belongs to the curved exponential family.

This approach yields to the following hypothesis:

- $p_0$ follows a probability distribution according to $\mathcal{N}\left(\overline{p_0}, \sigma_{p_0}^2\right)$, $\overline{p_0}$ and $\sigma_{p_0}^2$ being the mean and variance of the Gaussian distribution respectively,

- $t_0$ follows a probability distribution $\mathcal{N}\left(\overline{t_0}, \sigma_{t_0}^2\right)$, $\overline{t_0}$ and $\sigma_{t_0}^2$ being the mean and variance of the Gaussian distribution respectively,

- $v_0$ follows a probability distribution $\mathcal{N}\left(\overline{v_0}, \sigma_{v_0}^2\right)$, $\overline{v_0}$ and $\sigma_{v_0}^2$ being the mean and variance of the Gaussian distribution respectively,

- for all $k$, $\delta_k$ follows a probability distribution $\mathcal{N}\left(\overline{\delta_k}, \sigma_\delta^2\right)$, $\overline{\delta_k}$ and $\sigma_\delta^2$ being the mean and variance of the Gaussian distribution respectively, and

- matrix A is assumed to be writable as $A = \sum_{k=1}^{(N-1)N_S} c_k A_k$ where, for all $k$, $c_k$ following a probability distribution according to $\mathcal{N}\left(\overline{c_k}, \sigma_c^2\right)$, $\overline{c_k}$ and $\sigma_c^2$ being the mean and variance of the Gaussian distribution respectively, $A_k$ being an orthonormal basis of the sub-space in $T\{\gamma_\delta(t_0)\mathbb{M}\}$ that is orthogonal to $\dot{\gamma}(t_0)$ obtained using a Gram-Schmidt process. Under this hypothesis, the random variables $c_1, ..., c_{(N-1)NS}$ are considered hidden variables for the model. The previous hypothesis relative to matrix A ensures the orthogonality condition on the columns of A. The orthogonality condition ensures that a point on the parallel curve $\eta^{wi}(\gamma,.)$ moves at the same pace as in the average trajectory, so only the parameters of the time-reparameterization function accounts for the difference in the dynamics of the progression across the subjects. This property is crucial to ensure a unique decomposition between the spatial and temporal components, and thus the identifiability of the model.

Therefore, under the previous hypotheses, the vector parameter θ of the model is:

$$\boldsymbol{\theta} = \left(\overline{p_0}, \overline{t_0}, \overline{v_0}, \left(\overline{\delta_k}\right)_{1\leq k\leq N-1}, \left(\overline{c_k}\right)_{1\leq k\leq (N-1)Ns}, \sigma_\eta, \sigma_\tau, \sigma\right)$$

whereas the hidden variables z of the model are:

$$\boldsymbol{z} = \left(p_0, t_0, v_0, (\delta_k)_{1\leq k\leq N-1}, (c_k)_{1\leq k\leq (N-1)Ns}, (\eta_i)_{1\leq i\leq p}, (\tau_i)_{1\leq i\leq p}, (s_{j,i})_{1\leq j\leq N_S, 1\leq i\leq p}\right)$$

To obtain the vector parameter θ and hidden variables z, the MCMC-SAEM is iterated, until convergence, between three sub-steps: a first sub-step of simulation, a second sub-step of stochastic approximation and a third sub-step of maximization.

Let k be an integer greater than 1 and $\theta^{(k-1)}$ (respectively $\boldsymbol{z}^{(k-1)}$) denote the parameters (respectively the hidden variables) at the *(k-1)*-th iteration of the algorithm.

The **k**-th iteration can be described as follows.

At the sub-step of simulation, $z^{(k)}$ is sampled from the transition kernel of an ergodic Markov Chain whose stationary distribution is the conditional distribution of the hidden variables knowing the observations $\boldsymbol{y} = (y_{i,j})_{i,j}$ and the current estimates of the parameters $\theta^{(k-1)}$. This sampling is done by using a Hasting-Metropolis technique within a Gibbs sampler scheme.

At the sub-step of stochastic approximation, the stochastic approximation is done by calculating sufficent statistics as follows:

$$\mathbf{S}^{(k+1)} \leftarrow \mathbf{S}^{(k)} + \varepsilon_k(\mathbf{S}(\mathbf{y}, \mathbf{z}^{(k)}) - \mathbf{S}^{(k)})$$

where $(\varepsilon_k)_k$ is a decreasing sequence of positive step sizes.

In other words, the stochastic approximation sub-step consists in a stochastic approximation on the complete log-likelihood log q($\mathbf{y}, \mathbf{z} \mid \theta$) summarized as follows :

$$Q_t(\boldsymbol{\theta}) = Q_{t-1}(\boldsymbol{\theta}) + \varepsilon_t \left[\log q(\mathbf{y}, \mathbf{z} \mid \boldsymbol{\theta}) - Q_{t-1}(\boldsymbol{\theta})\right],$$

where $(\varepsilon_t)_t$ is a decreasing sequence of positive step-sizes in ]0, 1] which satisfies $\sum_t \varepsilon_t = +\infty$ and $\sum_t \varepsilon_t = +\infty$ and $\sum_t \varepsilon_t^2 < +\infty$ .

At the sub-step of maximization, parameters updates are obtained in closed form from the stochastic approximation on the sufficient statistics.

For instance, the parameter estimates are updated in the maximization step according to the following formula:

$$\Theta^{(t+1)} = \text{argmax}_{\theta \in \Theta}[Q_t(\boldsymbol{\theta})]$$

In summary, it has been disclosed a generic hierarchical spatiotemporal model for longitudinal manifold-valued data. The data consist in repeated measurements over time for a group of individuals. This numerical model NM enables estimating a group-average trajectory of progression, considered as a geodesic of a given Riemannian manifold. Individual trajectories of progression are obtained as random variations, which consist in parallel shifting and time reparametrization, of the average trajectory. These spatiotemporal transformations allow the applicant to characterize changes in the direction and in the pace at which trajectories are followed. The parameters of the model are estimated using a stochastic approximation of the expectation-maximization (EM) algorithm, the Monte Carlo Markov Chain Stochastic Approximation EM (MCMC SAEM) algorithm.

Experimental results obtained with the numerical model NM are illustrated in the experimental section.

Thus, at the end of the step of providing S32, both first data and a numerical model NM are obtained.

At the step of converting S34, the first data are converted into at least one point on the same Riemann manifold RM.

At the step of using S36, the numerical model NM is used to determine a temporal progression for the Alzheimer's disease for the studied subject.

These steps S34 and S36 are difficult to carry out so far as it requires to adapt (personalize) the parameters of the NM so that the trajectory of progression passes through, or as close as possible, to the first data converted as points on the Riemann manifold RM.

For instance, one may use the parameters of dispersion of the NM to generate series of trajectories on the Riemann manifold RM, which derives from the average trajectory by spatiotemporal transformations, such as the curves $C_1$ and $C_2$ in the example of Figure 4. The point(s) to which the first data correspond in the Riemann manifold RM are used to select the curve which minimizes a distance with these points.

If the distance with the first curve $C_1$ is the smallest, it is determined that the expected temporal progression for the Alzheimer's disease for the studied subject is the first curve $C_1$.

The present invention therefore enables to determine the temporal progression of a biological phenomenon based on data taken from a subject.

The present method provides a good versatility in so far as the Riemannian manifold RM and its metric are chosen a priori, which allows us to introduce anatomical, physiological constraints into the model. The definition of the generic spatiotemporal model requires no other choice. The models which are introduced herein are based on the concept of parallel curves on a manifold. The random effects of the model allow to spatially and temporally register individual trajectories of progression.

The absence of other choice required in the present invention is different from the prior art in which the model is constrained to fit the data. In other words, a reduced model (with hypothesis) is used to fit the data which means that prior to any fitting, the hypotheses are already known. On the contrary, the model is adapted in step S36 to the data and the obtained model enables to determine the hypotheses. In such context, the step S34 can be construed as a rescaling step of the parameters of the model so that the data be proximate. This rescaling is notably explained in the second and in the third experiments.

In addition, it should be stressed that the proposed method for determining is particularly easy to implement in so far as no constraints are imposed on the first data.

Given the complexity of the problem to address, it could have been indeed expected that constraints be imposed on the data to provide. In particular, there is no need to data taken at specific time points. As a specific example, there is no need to obtain data at a specific reference time, such as the date at which disease starts.

Furthermore, the present method for determining a temporal progression is usable in multiple applications.

For instance, such method for determining a temporal progression is used in a method for predicting that a subject is at risk of suffering from a disease.

The method for predicting comprises carrying out the steps of the method for determining the temporal progression of a biological phenomenon which may affect a studied subject, the biological phenomenon being the disease, to obtain a first temporal progression. The method for predicting also comprises predicting that the subject is at risk of suffering from the disease based on the first temporal progression.

According to a specific embodiment, the method for predicting further provides when specific symptom are expected to occur for the subject.

According to another example, the method for determining a temporal progression is used in a method for diagnosing a disease.

The method for diagnosing comprises carrying out the steps of the method for determining the temporal progression of a biological phenomenon which may affect a studied subject, the biological phenomenon being the disease, to obtain a first temporal progression. The method for diagnosing also comprises a step for diagnosing the disease based on the first temporal progression.

According to another example, the method for determining a temporal progression is used in a method for identifying a therapeutic target for preventing and/or treating a pathology.

The method for identifying a therapeutic target comprises carrying out the steps of the method for determining the temporal progression of a biological phenomenon which may affect a studied subject, the first data being data relative to a subject suffering from the pathology, to obtain a first temporal progression.

The method for identifying a therapeutic target also comprises carrying out the steps of the method for determining a temporal progression which may affect a studied subject, the first data being data relative to a subject not suffering from the pathology, to obtain a second temporal progression.

The method for identifying a therapeutic target also comprises a step of selecting a therapeutic target based on the comparison of the first and second temporal progressions.

In such context, the term « therapeutic target » should be construed broadly as encompassing selecting specific kind of patients.

According to yet another example, the method for determining is used in a method for identifying a biomarker.

The biomarker may vary according to the specific example considered. For instance, the biomarker is a diagnostic biomarker of a pathology. In variant, the biomarker is susceptibility biomarker of a pathology, a prognostic biomarker of a pathology or a predictive biomarker in response to the treatment of a pathology.

The method for identifying a biomarker comprises carrying out the steps of the method for determining the temporal progression of a biological phenomenon which may affect a studied subject, the first data being data relative to a subject suffering from the pathology, to obtain a first temporal progression.

The method for identifying a biomarker also comprises carrying out the steps of the method for determining the temporal progression of a biological phenomenon which may affect a studied subject, the first data being data relative to a subject not suffering from the pathology, to obtain a second temporal progression.

The method for identifying a biomarker also comprises a step of selecting a biomarker based on the comparison of the first and second temporal progressions.

According to another example, the method for determining is used in a method for screening a compound useful as a medicine.

The compound has an effect on a known therapeutical target, for preventing and/or modifying and/or treating a pathology.

The method for screening comprises carrying out the steps of the method for determining the temporal progression of a biological phenomenon which may affect a studied subject, the first data being data relative to a to a subject suffering from the pathology and having received the compound, to obtain a first temporal progression.

The method for screening also comprises carrying out the steps of the method for determining the temporal progression of a biological phenomenon which may affect a studied subject, the first data being data relative to a subject suffering from the pathology and not having received the compound, to obtain a second temporal progression.

The method for identifying a therapeutic target also comprises a step of selecting a therapeutic target based on the comparison of the first and second temporal progressions.

Each previously described application illustrate the possibility of the proposed method for determining a temporal progression.

EXPERIMENTAL SECTION: FIRST EXPERIMENT

The numerical model NM is used to analyze the temporal progression of a family of biomarkers. This progression model estimates a normative scenario of the progressive impairments of several cognitive functions, considered here as biomarkers, during the course of Alzheimer's disease. The estimated average trajectory provides a normative scenario of disease progression. Random effects provide unique insights into the variations in the ordering and timing of the succession of cognitive impairments across different individuals.

*Data considered*

The neuropsychological assessment tests "ADAS-Cog 13" from the ADNI1, ADNIGO or ADNI2 cohorts of the Alzheimer's Disease Neuroimaging Initiative (ADNI) was used. These tests are notably available at the internet address https://ida.loni.usc.edu/.

The "ADAS-Cog 13" consists of 13 questions, which allow testing the impairment of several cognitive functions. For the purpose of our analysis, these items are grouped into four categories: memory (5 items which are items 1, 4, 7, 8 and 9), language (5 items which are items 2, 5, 10, 11 and 12), praxis (2 items which are items 3 and 6) and concentration (1 item which is item 13).

248 individuals were included in the study. These 248 individuals were diagnosed with mild cognitive impairment at their first visit and the diagnosis changed to Alzheimer's disease before their last visit. There is an average of 6 visits per subjects, with an average duration of 6 or 12 months between consecutive visits. The minimum number of visits was 3 and the maximum number of visit was 11.

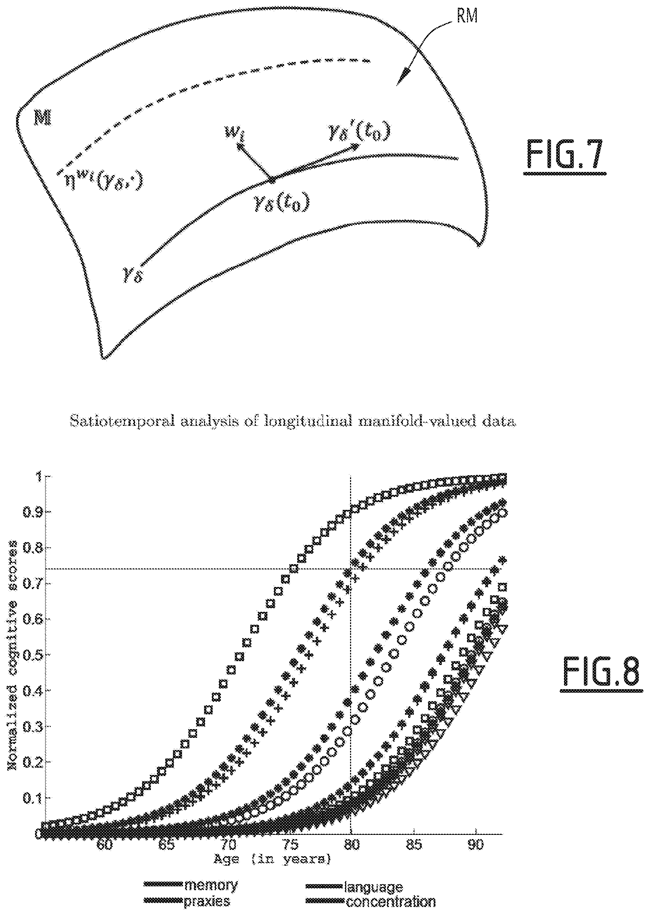

*For the experiments of Figures 8 to 12*

According to a first case, the score of each item was normalized by the maximum possible score. Consequently, each data point of each individual consists in thirteen normalized scores, which can be seen as a point on the manifold

$$\mathbb{M} = ]0, 1[^{13}.$$

In the case where M = ]0, 1[$^{13}$, the number of independent sources $N_s$ can be any integer between 1 and 12. The choice of the number of independent sources influences the number of parameters to be estimated, which equals 9 + 12*$N_s$. In order to keep a reasonable runtime, three experiments were conducted with $N_s$ equal to 1, 2 and 3. For each experiment, the MCMC-SAEM algorithm was run five times with different initial parameters. Only the experiment which returned the smallest residual noise variance was kept. Increasing the number of sources allowed to decrease the residual noise among the experiments : $\sigma^2$ =0.02 for $N_s$ = 1, $\sigma^2$ = 0.0162 for $N_s$ = 2 and $\sigma^2$ = 0.0159 for $N_s$ = 3. Because the residual noise was almost similar for $N_s$ = 2 and $N_s$ = 3 sources, the results obtained with the less complex model are described. As a consequence, the results obtained with two independent sources are further developed below.

The average trajectory $\gamma_\delta$ is given in Figure 8, where each curve represents the temporal progression of one specific item of the ADAS-Cog test. The estimated fixed effects are $p_0$ =0.74, $t_0$ = 79.88 years, $v_0$ = 0.047 unit per year, and in years:

$$\boldsymbol{\delta} = [0; -14; -11; 4.6; -13; -14; -7.7; -0.9; -14.4; -14.05; -11.80; -15.3292]$$

This means that, on average, the memory-related items (items 1, 4, 7, 8, 9) reach the value $p_0$ = 0.74 at respectively $t_0$, $t_0$-$\delta_4$, $t_0$-$\delta_7$, $t_0$-$\delta_8$ and t0-$\delta_9$ years, which correspond to respectively 79.88, 75.2, 87.6, 80.7 and 94.3 years. The concentration item reaches the same value at $t_0$-$\delta_{13}$ = 86.1 years. The progression of the concentration item is followed by praxis and language items.

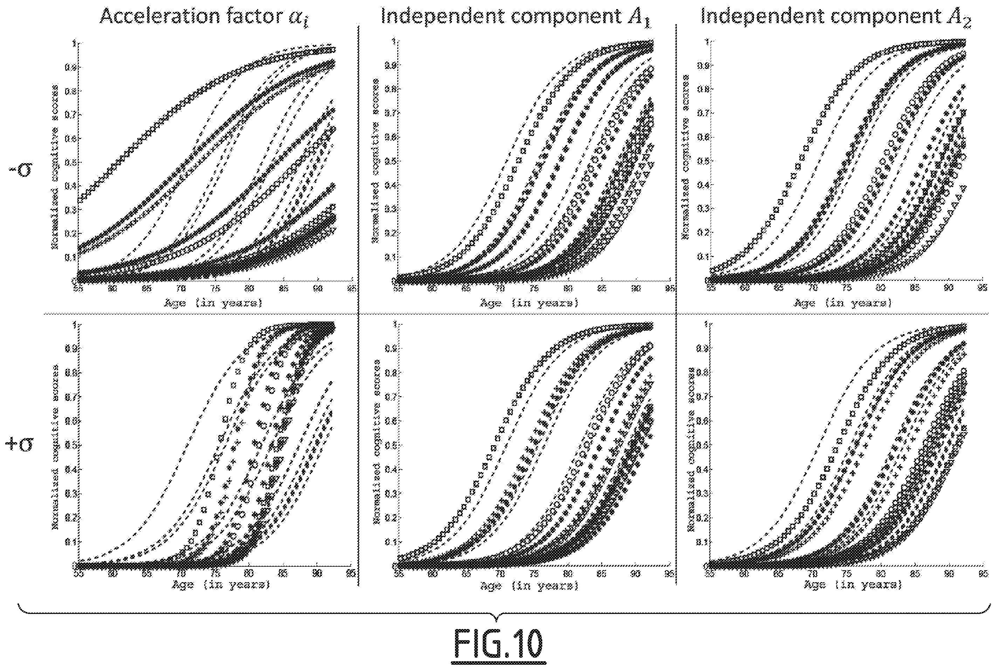

Random effects show the variability of this average trajectory within the studied population. The standard deviation of the time-shift equals $\sigma_\tau$ =8.3 years, meaning that the disease progression model in Figure 8 is shifted in time by at most $\pm$8.3 years for 95% of the population. This accounts for the variability in the age at disease onset among the population. The effects of the variance of the acceleration factors and the two independent components of the space-shifts are illustrated in Figure 10.

The first column of Figure 10 illustrates the variability in pace of disease progression (the time-shifts are assumed to be zero in order to illustrate the effect of acceleration factor only). This variability is encoded by the variance $\sigma_\eta$ =0.8 of the log-acceleration factor.

The first and second independent components illustrates the variability in the relative timing of the cognitive impairments.

The first independent direction shows that some memory items and language items are shifted in time with respect to the other ones, especially for memory item 4 ( ) and item 7 (∘). The ordering of the memory item 7 (∘) and the concentration item is inverted for individuals with a space shift $w_i$ = -$\sigma_{su,1}A_1$. For those individuals, praxies items are impaired later, after the language items 2(*), items 12 (Δ) and item 5 ( ).

The second independent component shows a greater variability for the memory-related items than for the first independent components, in particular for memory item 9 (Δ) and item 4 ( ). For individuals with a space shift $w_i$ = -$\sigma_{si,2}A_2$, language-related items might be impaired later than the average individual, especially for the language item 12 (Δ).

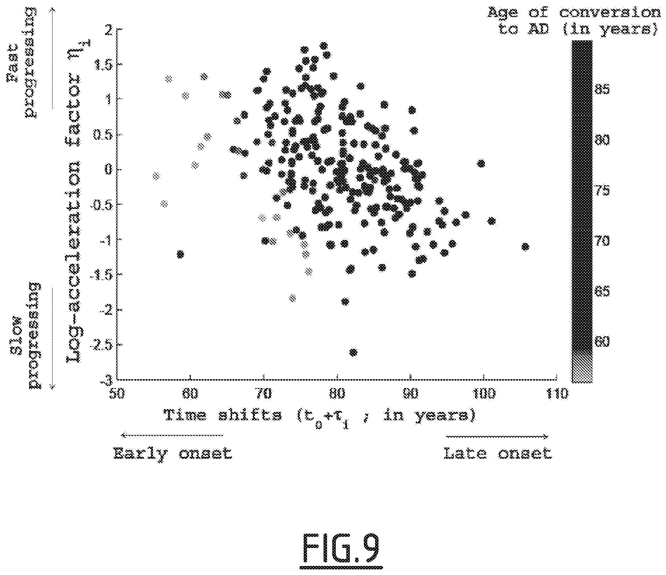

The subject-specific random effects estimated for each individual are obtained from the sampling step of the last iteration of the MCMC-SAEM and are plotted in Figure 9. The figure 9 shows that the individuals who have a positive (respectively negative) time shift (they are evolving ahead, respectively behind, the average trajectory) are the individuals who converted late (respectively early) to Alzheimer's disease. This means that the individual time-shifts correspond well to the age at which a given individual was diagnosed with Alzheimer's disease. It is also to be noted that there is a negative correlation, equal to -0.4, between the estimated log-acceleration factors and time shifts. There is a tendency for early onset patients to be fast progressers.

Through its subject-specific affine reparametrization, the age of a given individual is registered to the common timeline of the average scenario.

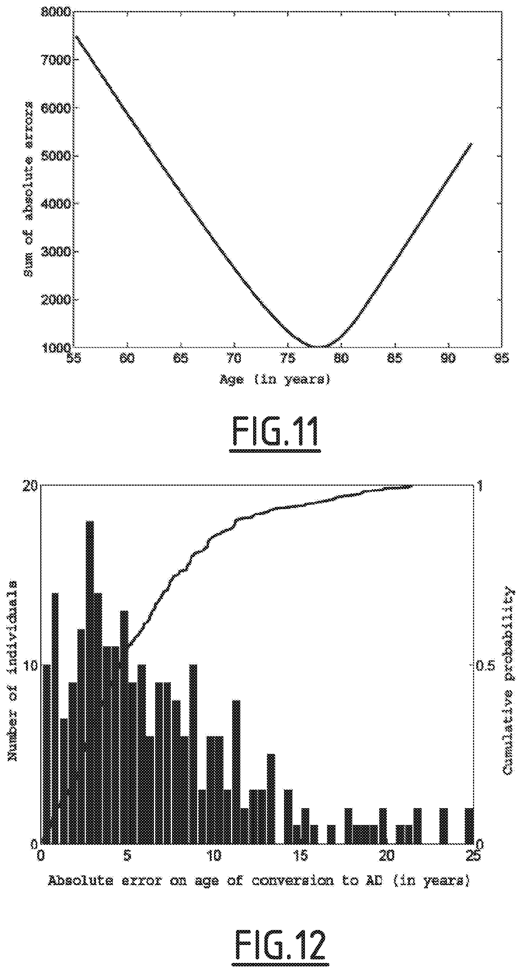

In Figure 11, the evolution of the sum of the absolute errors with time is represented. In this case, the sum of the absolute errors is $\sum_i \left| t_i^{diag} - \psi_i^{-1}(t) \right|$ where $t_i^{diag}$ corresponds to the age at which the i-th individual converted to Alzheimer's disease. As apparent on Figure 11, the sum of the absolute errors shows a unique minimum at t* = 77.45 years. This age can be understood as the age of symptoms onset in the timeline of the normative scenario of disease progression.

Figure 12 corresponds to a histogram giving the number of individuals with relation to the absolute errors on the age of conversion to Alzheimer's disease in years. In other words, the histogram of Figure 12 gives the number of individuals in function of the absolute error which is $\left| t_i^{diag} - \psi_i^{-1}(t*) \right|$ . The analysis of the histogram of Figure 12 shows that the age $\psi_i^{-1}(t*)$ is a prediction of the true age of conversion : the error of prediction is less than 5 years for 50% of the population. This prediction is all the more remarkable as this prediction is obtained by analyzing cognitive scores, which are inherently noisy and whose reproducibility is limited.

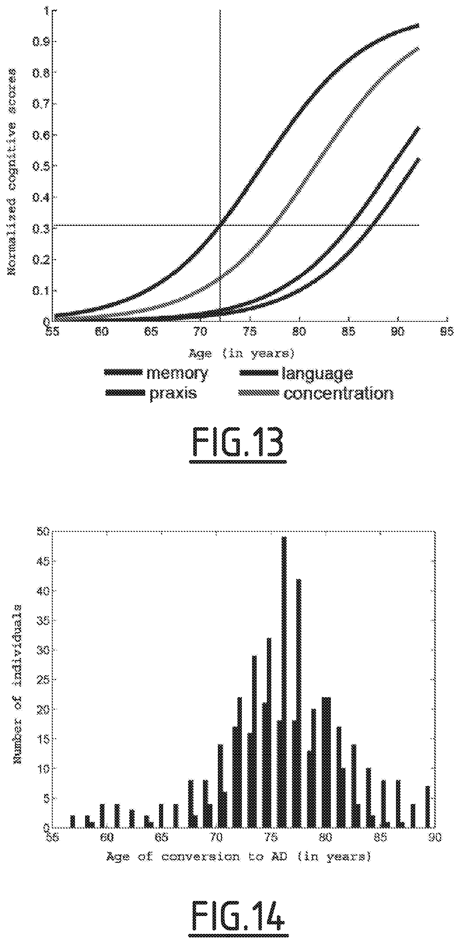

*For the experiments of Figures 13 to 16*

According to a second case, scores within each category are added and normalized by the maximum possible score. Consequently, each data point consists in four normalized scores, which can be seen as a point on the manifold $\mathbb{M} = ]0, 1[^4$ .

The model was applied with $N_s$ = 1, 2 or 3 independent sources. In each experiment, the MCMC SAEM was run five times with different initial parameter values. The experiment which returned the smallest residual variance $\sigma^2$ was kept. The maximum number of iterations was arbitrarily set to 5000 and the number of burn-in iterations was set to 3000 iterations. The limit of 5000 iterations is enough to observe the convergence of the sequences of parameters estimates. As a result, two and three sources allowed to decrease the residual variance better than one source ($\sigma^2$ = 0.012 for one source, $\sigma^2$ = 0.08 for two sources and $\sigma^2$ = 0.084 for three sources). The algorithm was implemented in MATLAB® without any particular optimization scheme. The 5000 iterations required approximately one day.

The number of parameters to be estimated was equal to 9+3*$N_s$. Therefore, the number of sources did not dramatically impact the runtime. Simulation was the most computationally expensive part of the algorithm. For each run of the Hasting-Metropolis algorithm, the proposal distribution was the prior distribution.

For a matter of clarity and because the results obtained with three sources were similar to the results with two sources, the experimental results obtained with two independent sources are further detailed.

The average model of disease progression $\gamma_\delta$ is plotted on Figure 13. Figure 13 is a graph showing the evolution with the age of the memory, language, praxis and concentration in terms of normalized cognitive score. The four curves represented correspond to the estimated average trajectory. A vertical line is drawn at $t_0$ = 72 years old and a horizontal line is drawn at $p_0$ = 0.3.

When analyzing Figure 13, it appears that the estimated fixed effects are $p_0$ =0.3, $t_0$ = 72 years, $v_0$ =0.04 unit per year, and $\delta$ = [0; -15; -13; -5] years. This means that, on average, the memory score (first coordinate) reaches the value $p_0$ at $t_0$ = 72 years, followed by concentration which reaches the same value at $t_0$ +5 = 77 years, and then followed by praxis and language at the age of 85 and of 87 years respectively.

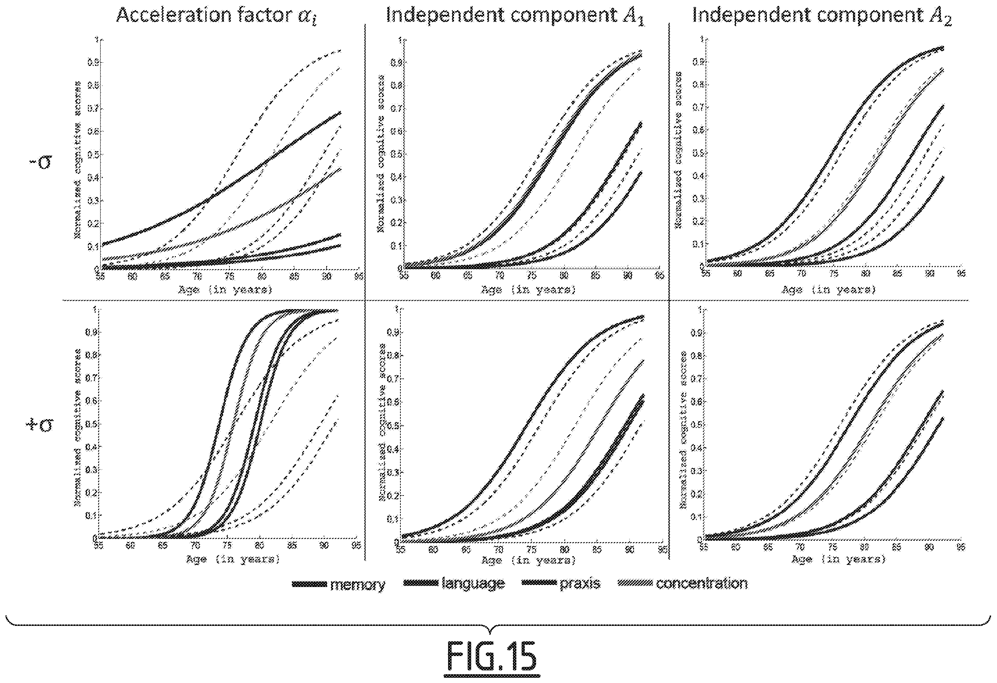

Random effects show the variability of this average trajectory within the studied population. The standard deviation of the time-shift equals $\sigma_\tau$ =7.5 years, meaning that the disease progression model in Figure 13 is shifted by $\pm$7.5 years to account for the variability in the age of disease onset. The effects of the variance of the acceleration factor $\alpha_i$, and the two independent components $A_1$ and $A_2$ of the space-shifts are illustrated in Figure 15.

Figure 15 is a series of six graphs illustrating the variability in disease progression superimposed with the average trajectory $\gamma_\delta$ (dotted lines).

On the first column, the effects of the acceleration factor $\alpha_i$ with plots of $\gamma_\delta$ (exp($\pm\sigma_\eta$)(t - $t_0$)+ $t_0$) are represented. The acceleration factor $\alpha_i$ shows the variability in the pace of disease progression, which ranges between 7 times faster and 7 times slower than the average.

On the second column, the effects of the first independent component of space-shift with plots of $\eta^{\pm\sigma_{s_1}, A_1}\left(\gamma_\delta, .\right)$ are illustrated. The first independent component $A_1$ shows variability in the relative timing of the cognitive impairments: in one direction, memory and concentration are impaired nearly at the same time, followed by language and praxis; in the other direction, memory is followed by concentration and then language and praxis are nearly superimposed.

On the third column, the effects of the first independent component of space-shift with plots of $\eta^{\pm\sigma_{s_2}, A_2}\left(\gamma_\delta, .\right)$ are represented. The second independent component $A_2$ keeps almost fixed the timing of memory and concentration, and shows a great variability in the relative timing of praxis and language impairment. It shows that the ordering of the last two may be inverted in different individuals. Overall, these space-shift components show that the onset of cognitive impairment tends to occur by pairs: memory and concentration followed by language and praxis.

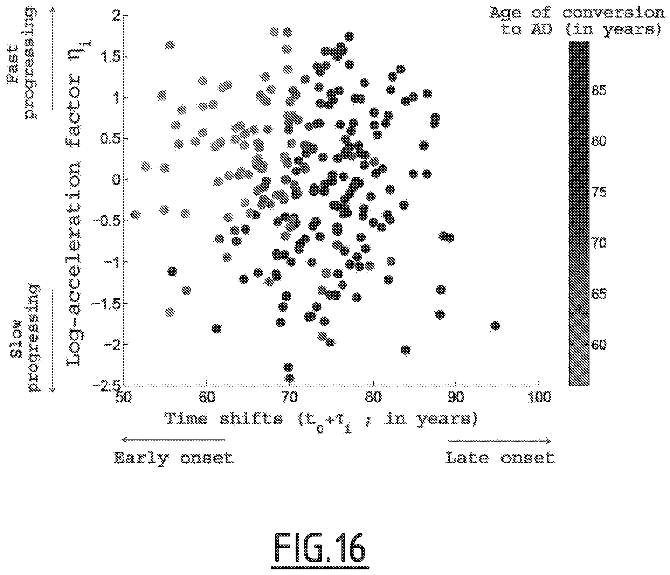

Estimates of the random effects for each individual are obtained from the simulation step of the last iteration of the algorithm and are plotted in Figure 16. In a similar way to Figure 9, Figure 16 shows that the estimated individual time-shifts correspond well to the age at which individuals were diagnosed with Alzheimer's disease. This means that the value $p_0$

estimated by the model is a good threshold to determine diagnosis (a fact that has occurred by chance), and more importantly that the time-warp correctly registers the dynamics of the individual trajectories so that the normalized age correspond to the same stage of disease progression across individuals. This fact is corroborated by Figure 14 which shows that the normalized age of conversion to Alzheimer's disease is picked at 77 years old with a small variance compared to the real distribution of age of conversion. Figure 14 is a histogram diagram of the ages of conversion to Alzheimer's disease with time-warp (grey bars) and the normalized ages of conversion to Alzheimer's disease (black bars).

EXPERIMENTAL SECTION: SECOND EXPERIMENT

*Data*

We used this model to highlight typical spatiotemporal patterns of cortical atrophy during the course of Alzheimer's Disease from longitudinal MRI of MCI converters from the ADNI database. This 154 MCI converters corresponding to 787 observations, each subject being observed 5 times on average. To get a common fixed-graph, we aligned the measures on a common atlas with FreeSurfer (which is publicly available notably at http://surfer.nmr.mgh.harvard.edu) in order to distribute the measurements maps on the same underlying graph G, constituted of 1827 nodes corresponding to areas uniformly distributed over the brain surface. Out of these vertices, the Applicant selected 258 control nodes that encode the spatial interpolation of the propagation. The distance matrix D used is defined by a geodesic distance on the graph G.

*Cortical thickness measurements*

The Applicant used the model instantiation defined in the next paragraph to characterize the cortical thickness decrease. Multiple runs of 30.000 iterations (~4 hours) of this 1827 dimensional MCMC-SAEM lead to a noise standard deviation $\sigma$ equal to 0.27 with 90% of the data included in the segment [1.5,3.6].
To represent this propagation, the Applicant computed the thickness difference over 5 year periods. The mean spatiotemporal propagation as the cortical thickness decrease between 80 and 90 years old, shows that the most affected area is the medial-temporal lobe, followed by the temporal neocortex. The parietal association cortex and the frontal lobe are also subject to important alterations. On the other side, the sensory-motor cortex and the visual cortex are less involved in the lesion propagation. These results are highly consistent with previous knowledge of the Alzheimer's Disease effects on the brain structure. As the model is able to exhibit individual spatiotemporal patterns with their associated pace of progression, it respectively refer to individuals with a faster and slower propagation of the disease. The thickness decrease is thus respectively more and less substantial than the mean scenario.

*Model instantiation*

As many measurements correspond to positive values (eg. the cortical thickness, volume ratios), the Applicant considers in the following the open interval $M = ]0,+\infty[$ as a one-dimensional Riemannian manifold equipped with a Riemannian metric $g$ such that for all $p \in M$ and for all $(u,v) \in T_p M$, $g_p(u,v) = uv/p$. With this metric and given $k \in \{1,...,N_v\}$, $M$ is a geodesically complete Riemannian manifold whose geodesics are of the form

$t \to p_k \exp(v_k/p_k*(t - t_0 - \delta_k))$ with $p_k \in M$, $t_0$, $\delta_k \in \mathbb{R}$ and $v_k \in T_{pk}M$.
For identifiability reasons, we choose to fix the parameter $p_k$ among the nodes, leading to a shared parameter $p_0$. Considering the interpolation functions introduced previously and the fact that the parameters ($p_j^k$) are $(\delta_k, v_k)$, it leads to define:

$$\delta(x) = \sum_{i=1}^{N_C} K(x, x_d)\, \beta_\delta^i \text{ and } v(x) = \sum_{i=1}^{N_C} K\left(x, x_{d_i}\right) \beta_v^i$$

The model can be rewritten as:

$$(y_{i,j})_k = p_0 exp\left(\frac{(w_i)_k}{p_0 exp\left(\frac{v(x_k)}{p_0}\delta(x_k)\right)} + \frac{v(x_k)}{p_0}\delta(x_k) + \frac{v(x_k)}{p_0}\alpha_i\left(t_{i,j} - t_0 - \tau_i\right)\right) + (\epsilon_{i,j})_k$$

such that $\theta = \left(p_0, t_0, \left(\beta_\delta^i\right)_{1 \leq i \leq N_C}, \left(\beta_v^i\right)_{1 \leq i \leq N_C}, \sigma\right)$ and $z = (w_i, \alpha_i, \tau_i)_{1 \leq i \leq}p$.

*Discussion and perspectives*

It has been proposed a mixed-effect model which is able to evaluate a group-average spatiotemporal propagation of a signal at the nodes of a mesh thanks to longitudinal neuroimaging data distributed on a common network. The network edges describe the evolution of the signal whereas its vertices encode a distance between the nodes via a distance matrix. The high dimensionality of the problem is tackled by the introduction of control nodes: they allow to evaluate a smaller number of parameters while ensuring the smoothness of the signal propagation through neighbour nodes. Moreover, individual parameters characterize personalized patterns of propagation as variations of the mean scenario.

The evaluation of this non-linear high dimensional model is made with the MCMC-SAEM algorithm that leads to convincing results: we were able to highlight areas affected by considerable neuronal loss such as the medial-temporal lobe or the temporal neocortex.

The distance matrix, which encodes here the geodesic distance on the cortical mesh, may be changed to account for the structural or functional connectivity information.

In this case, signal changes may propagate not only across neighbouring locations, but also at nodes far apart in space but close to each other in the connectome.

This model could also be used with multimodal data, such at PET scans, introducing numerical models of neurodegenerative diseases. These models could first inform about the evolution of a disease at a population level while being customizable to fit individual data, thus predicting stage of the disease or time to symptom onset.

EXPERIMENTAL SECTION: THIRD EXPERIMENT

In the experiment, the Applicant proposes a method to predict the subject-specific longitudinal evolution of brain structures extracted from baseline MRI, and evaluate its performance on Alzheimer's disease data. The disease progression is modeled as a trajectory on a group of diffeomorphisms in the context of large deformation diffeomorphic metric mapping. The Applicant first exhibits the limited predictive abilities of geodesic regression extrapolation on this group. Building on the recent concept of parallel curves in shape manifolds, the Applicant introduces a second predictive protocol which personalizes previously learned trajectories to new subjects, and investigate the relative performances of two parallel shifting paradigms. This design only requires the baseline imaging data. Finally, coefficients encoding the disease dynamics are obtained from longitudinal cognitive measurements for each subject, and exploited to refine our methodology which is demonstrated to successfully predict the follow-up visits.

*Introduction*

The primary pathological developments of a neurodegenerative disease such as Alzheimer's are believed to spring long before the first symptoms of cognitive decline. Subtle gradual structural alterations of the brain arise and develop along the disease course, in particular in the hippocampi regions, whose volumes are classical biomarkers in clinical trials. Among other factors, those transformations ultimately result in the decline of cognitive functions, which can be assessed through standardized tests. Being able to track and predict future structural changes in the brain is therefore key to estimate the individual stage of disease progression, to select patients in clinical trials, and to assess treatment efficacy.

To this end, the Applicant's work settles down to predict the future shape of brain structures segmented from MRIs. The Applicant propose a methodology based on three building blocks : extrapolate from the past of a subject ; transfer the progression of a reference subject observed over a longer time period to new subjects ; and refine this transfer with information about the relative disease dynamics extracted from cognitive evaluations. Instead of limiting ourselves to specific features such as volumes, we propose to see each observation of a patient at a given time-point as an image or a segmented surface mesh in a shape space.

In computational anatomy, shape spaces are usually defined via the action of a group of diffeomorphisms. In this framework, one may estimate a flow of diffeomorphisms such that a shape continuously deformed by this flow best fits repeated observations of the same subject over time, thus leading to a subjectspecific spatiotemporal trajectory of shape changes. If the flow is geodesic in the sense of a shortest path in the group of diffeomorphisms, this problem is called geodesic regression and may be thought of as the extension in Riemannian manifolds of the linear regression concept. It is tempting then to use such regression to infer the future evolution of the shape given several past observations. To the best of our knowledge, the predictive power of such a method has not yet been extensively assessed. The Applicant will demonstrate that satisfying results can only be obtained when large numbers of data points over extensive periods of time are available, and that poor ones should be expected in the more interesting use-case scenario of a couple of observations.

In such situations, an appealing workaround would be to transfer previously acquired knowledge from another patient observed over a longer period of time. This idea requires the definition of a spatiotemporal matching method to transport the trajectory of shape changes in a different subject space. Several techniques have been proposed to register image time series of different subjects. They often require time series to have the same number of images, or to have correspondences between images across time series, and are therefore unfit for prognosis purposes. Parallel transport in group of diffeomorphisms have been recently introduced to infer deformation of follow-up images from baseline matching. Such paradigms have been used mostly to transport spatiotemporal trajectories to the same anatomical space for hypothesis testing]. We build on this concept to infer the subject trajectory continuation in the future. Two main methodologies have emerged : either by transporting the time series parallel to the baseline matching, or by transporting the baseline matching parallel to the time series as in the present invention. Both methods are evaluated in this section.

In any case, these approaches require to match the baseline shape with one in the reference time series. Ideally, we should match observations corresponding to the same disease stage, which is unknown. The Applicant proposes to complement such approaches with estimates of the patient stage and pace of progression using repeated neuropsychological assessments of the subjects, in the spirit of this invention. These estimates may be used to adjust the dynamics of shape changes of the reference subject to the test subject, according to the dynamical differences observed in the cognitive tests.

*Method*

Let $(y_i)_{i=1,\ldots,n}$ be a time series of segmented surface meshes for a given subject, obtained at the ages $(t_i)_{i=1,\ldots,n}$. The Applicant builds a group of diffeomorphisms of the ambient space which act on the segmented meshes, following the procedure described in the prior art. Flows of diffeomorphisms of $\mathbb{R}^3$ are generated by integrating time-varying vector fields of the form $v(t,x) = \sum_{i=1}^{N} K\big(x, c_k(t)\big)\beta_k(t)$ where:

- K is a Gaussian kernel,
- $(c_k(t))_{i=1,\ldots,N}$ are control points of the deformation, and
- $(\beta_k(t))_{i=1,\ldots,N}$ are the momenta of the deformation.

The space of diffeomorphisms is endowed with a norm which measures the cost of the deformation. In the following, are only considered geodesic flows of diffeomorphisms i.e. flows of minimal norm connecting the identity to a given diffeomorphism. Such flows are uniquely parametrized by their initial control points and momenta $(c^0, \beta^0)$. Under the action of the flow of diffeomorphisms, an initial template shape $T$ is continuously deformed and describes a trajectory in the shape space, which the Applicant will note $\gamma_{(c0,\beta0)}(T,.)$. Simultaneously, the surface meshes are endowed with a varifold norm $\|.\|$ which allows to measure a data attachment term between meshes without point correspondence.

*Geodesic regression*

In the spirit of linear regression, one can perform geodesic regression in the shape space by estimating the intercept $T$ and the slope $(c^0, \beta^0)$ such that $\gamma_{(c0,\beta0)}(T,.)$ minimizes the following functional :

$$\inf_{c^0, \beta^0, T} \sum_{i=1}^{N} \left\| \gamma_{(c^0, \beta^0)}(T, t_i) - y_i \right\|^2 + R(c^0, \beta^0)$$

where R is a regularization term which penalizes the kinetic energy of the deformation. A solution of equation (1) is estimated with a Nesterov gradient descent as implemented in the software Deformetrica (www.deformetrica.org), where the gradient with respect to the control points, the momenta and the template is computed with a backward integration of the data attachement term along the geodesic. Solving this optimisation problem gives a description of the progression of the brain structures which lies in the tangent space at the identity of the group of diffeomorphisms.

Once an optimum is found, the Applicant can extrapolate the geodesic to further ages and attempt to predict the future evolution of the brain structures.

*Two methods to transport spatiotemporal trajectories of shapes*

As it will be demonstrated later, geodesic regression extrapolation produces an accurate prediction only if data over a long time span is available for the subject, which is not compatible with the ambition of early prognosis.

Given a reference geodesic, the Riemannian parallel transport is used to generate a new trajectory. It is first performed a baseline matching between the reference subject and the new subject, which can be described as a vector in the tangent space of the group of diffeomorphisms. Two paradigms are available to obtain a parallel trajectory. It is known in prior art to transport the reference regression along the matching and then shoot. In the shape space, this generates a geodesic starting at the baseline shape ; for this reason, this solution is called *geodesic parallelization.* On the other hand, in the present invention, it is suggested to transport the matching vector along the reference geodesic and then build a trajectory with this transported vector from every point of the reference geodesic. This procedure is called *exp-parallelization.*

In such a high-dimensional setting, the computation of parallel transport very often relies on the Schild's ladder scheme. However, in the Applicant's case the computation of the Riemannian logarithm may only be computed by solving a shape matching problem, resulting not only in an intractable algorithm but also in an uncontrolled approximation of the scheme. To implement these parallel shifting methods, it was used an algorithm which relies on an approximation of the transport to nearby points by a well-chosen Jacobi field, with a sharp control on the computational complexity. The same rate of convergence as Schild's ladder is obtained at a reduced cost.

*Cognitive scores dynamics*

The protocol described in the previous section has two main drawbacks. First, the choice of the matching time in the reference trajectory is arbitrary : the baseline is purely a convenience choice and ideally the matching should be performed at similar stages of the disease. Second, it does not take into account the pace of progression of the subject. In the present invention, the Applicant proposes a statistical model allowing to learn, in an unsupervised manner, dynamical parameters of the subjects from ADAS-cog test results, a standardized cognitive test designed for disease progression tracking. More specifically, they suppose that each patient follows a parallel to a mean trajectory, with a time reparametrization :

$$\psi(t) = \alpha \ (t - t_0 - \tau) + t_0 \ (2)$$

which maps the subject time to a normalized time frame, where $\alpha > 0$ and $\tau$ are scalar parameters. A high (respectively low) value of a hence corresponds to a fast (respectively slow) progression of the scores. With these dynamical parameters, the shape evolution can be adjusted by reparametrizing the parallel trajectory in the same way.

<u>*Results*</u>

*Data, preprocessing, and global parameters*

MRIs are extracted from the ADNI database, where only MCI converters with 7 or more visits are kept, for a total of N=74 subjects and 634 visits. Subjects are observed for a period of time ranging from 4 to 9 years (5.9 on average), with 12 visits at most.

The 634 MRIs are segmented using the FreeSurfer software. The extracted brain masks are then affinely registered towards the Colin 27 Average Brain using the FSL software. The estimated transformations are finally applied to the pairs of caudates, hippocampi and putamina subcortical structures.

All diffeomorphic operations i.e. matching, geodesic regression estimation, shooting, exp-parallelization and geodesic parallelization are performed thanks to the Deformetrica software previously mentionned. A varifold distance with Gaussian kernel width of 3 mm for each structure and a deformation kernel width of 5 mm are chosen. The time discretization resolution is set to 2 months.

*Geodesic regression extrapolation*

The acceleration factor $\alpha$ in equation (2) encodes the rate of progression of each patient. Multiplying this coefficient with the actual observation window gives a notion of the absolute observation window length, in the disease time referential. Only the 22 first subjects according to this measure have been considered for this section : they are indeed expected to feature large structural alterations, making the geodesic regression procedure more accurate.

Table 1 presents the results obtained for varying learning dataset and extrapolation extents.

The performance metric between two sets of meshes is the Dice coefficient, that is the sum of the volumes of the intersections of the corresponding meshes, divided by the total sums of the volumes. It is comprised between 0 and 1 : it equals 1 for a perfect match, and 0 for disjoint structures. The geodesic regression predictive performance is compared to a reference one consisting of the last observed brain structures in the learning dataset. A Mann-Whitney test was performed with the null hypothesis that the observed Dice coefficients distributions are the same to obtain the statistical significance levels.

The extrapolated meshes are satisfying only in the case where all but one data points are used to perform the geodesic regression, achieving a high Dice index and outperforming the reference one, by a small margin though and failing to reach the significance level ($p = 0.25$). When the window of observation becomes narrower, the prediction accuracy decreases and becomes worse than the reference one.

*Table 1: Averaged Dice performance measures*

| Learning period (months) | Method | Predicted follow-up visit | | | | | |
|---|---|---|---|---|---|---|---|
| | | M12 N=22 | M24 N=21 | M36 N=19 | M48 N=18 | M72 N=16 | M96 N=5 |
| 6 | [reg] | .878 | .800 | .737 | .624 | .509 | .483 |
| | [ref] | .888 | .850 | .803 | .708 | .626 | .602 |
| 12 | [reg] | - | .839 | .769 | .658 | .523 | .465 |
| | [ref] | - | .875 | .832 | .735 | .644 | .608 |
| 18 | [reg] | - | .855 | .823 | .738 | .611 | .579 |
| | [ref] | - | .890 | .851 | .764 | .661 | .627 |
| 24 | [reg] | - | - | .864 | .778 | .681 | .657 |
| | [ref] | - | - | .869 | .779 | .689 | .653 |
| Max -1 about 60 months | [reg] | .807 | Prediction at the most remote possible time (about 6 months) for all subjects (N = 22) | | | | |
| | [ref] | .797 | | | | | |

*The [reg] tag indicates the regression-based prediction, and [ref] the reference one. Each row corresponds to an increasingly large learning dataset, patients being observed for widening periods of time. Each column corresponds to an increasingly remote predicted visit from baseline. Significance levels [.05, .01, .001, .0001].*

*Non reparametrized transport*

Among the 22 subjects whose regression-based predictive power has been evaluated in the previous section, the two which performed best are chosen as references for the rest of this paper. Their progressions are transported onto the 73 other subjects with the two different parallel shifting methods. We obtain a total of 288 predicted spatiotemporal trajectories - a few visits were excluded for they fatally failed at some stage of the processing pipeline, for meaningless reasons. In more details, for each pair of reference and target subjects, the baseline target shape is first registered to the reference. The reference geodesic regression is then either exp-parallelized or geodesically parallelized. Prediction performance is then assessed : the Dice index between the prediction and the actual observation, for the two modes of transport, are computed and compared to the Dice index between the baseline meshes and the actual observation - the only available information in the absence of a predictive paradigm.

The upper part of table 2 presents the results. In most cases, the obtained meshes by the proposed protocol are of lesser quality than the reference ones, according to the Dice performance metric. The two methods of transport are essentially similarly predictive, although geodesic parallelization slightly outperforms the exp-parallelization for the M12 prediction.

*Table 2: Averaged Dice performance measures*

| Time reparam. | Method | Predicted follow-up visit | | | | | |
|---|---|---|---|---|---|---|---|
| | | M12 | M24 | M36 | M48 | M72 | M96 |
| Without reparam. | | N=144 | N=138 | N=129 | N=76 | N=22 | N=11 |
| | [exp] | .878 | .841 | .799 | .650 | .626 | .647 |
| | [geod] | .883 | .847 | .806 | .664 | .523 | .661 |
| | [ref] | .882 | .850 | .806 | .682 | .644 | .611 |
| With reparam. | | N=140 | N=134 | N=123 | N=113 | N=62 | N=17 |
| | [exp] | .882 | .852 | .825 | .796 | .756 | .730 |
| | [geod] | .888 | .858 | .831 | .802 | .762 | .732 |
| | [ref] | .884 | .852 | .809 | .764 | .706 | .636 |

*In each cell, the first line gives the average Dice of the exp-parallelization-based prediction [exp], the middle line the geodesic parallelization-based one [geod], and the last line the reference Dice [ref]. Each column corresponds to an increasingly remote predicted visit from baseline. Signicance levels [.05, .01, .001, .0001].*

*Refining with cognitive dynamical parameters*

At the exception of the M12 prediction, both protocols consistently outperform the reference. The M36, M48, M72 and M96 predictions are the most impressive ones, with p-values always lesser than 1%. This shows that the pace of cognitive score evolution is well correlated with the pace of structural brain changes, and therefore allows an enhanced prediction of follow-up shapes.

No conclusion can be drawn concerning the two parallel shifting methodologies, a single weak significance result being obtained for the M12 prediction.

<u>Conclusion</u>

The Applicant conducted a quantitative study of geodesic regression extrapolation, exhibiting its limited predictive abilities. The Applicant then proposed a method to transport a spatiotemporal trajectory into a different subject space with cognitive declinederived time reparametrization, and demonstrated its potential for prognosis. The results show how crucial the dynamics are in disease modeling, and how cross-modality data can be exploited to improve a learning algorithm. The two main paradigms that have emerged for the transport of parallel trajectories were shown to perform equally well in this prediction task. Nonetheless, given the importance of the time-reparameterization, the exp-parallelization offers a methodological advantage in that it can be more easily combined with such reparameterization in generative statistical model for longitudinal data.

The robustness of the proposed protocol to the choice of reference subject has not been assessed. Such a choice could be avoided by constructing an average disease model. This framework may also be used to estimate a joint image and cognitive model to better estimate individual dynamical parameters of disease progression.

**Claims**

1. A computer-implemented method for determining the temporal progression of a biological phenomenon which may affect a studied subject, the biological phenomenon being a disease or ageing, the method comprising the steps of:

    - providing first data, the first data being biomarkers of the progression of the disease or ageing for the studied subject,
    - providing a numerical model (NM), the numerical model (NM) being a function in a Riemann manifold (RM), the numerical model (NM) associating to values of biomarkers a temporal progression trajectory for the biological phenomenon and the dispersion of the progression trajectory for the biological phenomenon among a plurality of subjects, the numerical model (NM) being obtained by using a stochastic approximation in an expectation-maximization technique on biomarkers taken at different time points for a plurality of subjects,
    - converting the first data into at least one point on the same Riemann manifold (RM), and

- using the numerical model (NM) on the at least one point obtained at the converting step to determine a temporal progression for the biological phenomenon for the studied subject.

2. The method according to claim 1, wherein the biological phenomenon is a biological phenomenon whose temporal progression extends over more than three years.

3. The method according to claim 1 or 2, wherein the biological phenomenon is a neurodegenerative disease.

4. The method according to any one of claims 1 to 3, wherein, at the step of providing first data, the first data are neuropsychological biomarkers.

5. The method according to any one of claims 1 to 4, wherein the studied subject is an animal, preferably a mammal, more preferably a human being.

6. The method according to any one of claims 1 to 5, wherein at the step of providing the numerical model (NM), the stochastic approximation in an expectation-maximization technique is a Monte-Carlo Markov Chain Stochastic Approximation Expectation-Maximization technique.

7. The method according to any one of claims 1 to 6, wherein at the step of providing the numerical model (NM), the number of subjects is superior or equal to 100.

8. The method according to any one of claims 1 to 7, wherein, in the numerical model (NM), the dispersion of the progression trajectory for the biological phenomenon among a plurality of subjects are provided as standard deviations of time for a plurality of values of biomarkers.

9. A method for predicting that a subject is at risk of suffering from a disease, the method for predicting at least comprising the step of:

   - carrying out the steps of the method for determining the temporal progression of a biological phenomenon which may affect a studied subject to any one of claims 1 to 8, the biological phenomenon being the disease, to obtain a first temporal progression, and
   - predicting that the subject is at risk of suffering from the disease based on the first temporal progression.

10. A method for diagnosing a disease, the method for diagnosing at least comprising the step of:

    - carrying out the steps of the method for determining the temporal progression of a biological phenomenon which may affect a studied subject to any one of claims 1 to 8, the biological phenomenon being the disease, to obtain a first temporal progression, and
    - diagnosing the disease based on the first temporal progression.

11. Method for identifying a biomarker, the biomarker being a diagnostic biomarker of a pathology, a susceptibility biomarker of a pathology, a prognostic biomarker of a pathology or a predictive biomarker in response to the treatment of a pathology, the method comprising the steps of:

    - carrying out the method for determining the temporal progression of a biological phenomenon which may affect a studied subject according to any one of claims 1 to 8, the first data being data relative to a subject suffering from the pathology, to obtain a first temporal progression,
    - carrying out the method for determining the temporal progression of a biological phenomenon which may affect a studied subject according to any one of claims 1 to 8, the first data being data relative to a subject not suffering from the pathology, to obtain a second temporal progression,
    - selecting a biomarker based on the comparison of the first and second temporal progressions.

12. A computer program product comprising instructions for carrying out the steps of a method according to any one of claims 1 to 11 when said computer program product is executed on a suitable computer device.

13. A computer readable medium having encoded thereon a computer program product according to claim 12.

**Patentansprüche**

1. Computerimplementiertes Verfahren zum Bestimmen des zeitlichen Verlaufs eines biologischen Phänomens, das ein untersuchtes Subjekt betreffen kann, wobei das biologische Phänomen eine Krankheit oder Altern ist, das Verfahren umfassend die folgenden Schritte:

   - Bereitstellen erster Daten, die Biomarker für den Verlauf der Krankheit oder des Alterns des untersuchten Subjekts sind,
   - Bereitstellen eines numerischen Modells (NM), wobei das numerische Modell (NM) eine Funktion in einer Riemannschen Mannigfaltigkeit (RM) ist, wobei das numerische Modell (NM) mit Werten von Biomarkern eine zeitliche Verlaufstrajektorie für das biologische Phänomen und die Streuung der Verlaufstrajektorie für das biologische Phänomen unter einer Vielzahl von Subjekten assoziiert, wobei das numerische Modell (NM) durch Verwenden einer stochastischen Annäherung in einer Erwartungsmaximierungstechnik an Biomarkern, die zu verschiedenen Zeitpunkten für eine Vielzahl von Subjekten genommen wurden, erlangt wird,
   - Konvertieren der ersten Daten in mindestens einen Punkt auf derselben Riemannschen Mannigfaltigkeit (RM), und
   - Verwenden des numerischen Modells (NM) auf den mindestens einen im Umwandlungsschritt erlangten Punkt, um einen zeitlichen Verlauf des biologischen Phänomens für das untersuchte Subjekt zu bestimmen.

2. Verfahren nach Anspruch 1, wobei das biologische Phänomen ein biologisches Phänomen ist, dessen zeitlicher Verlauf sich über mehr als drei Jahre erstreckt.

3. Verfahren nach Anspruch 1 oder 2, wobei das biologische Phänomen eine neurodegenerative Krankheit ist.

4. Verfahren nach einem der Ansprüche 1 bis 3, wobei die ersten Daten in dem Schritt eines Bereitstellens der ersten Daten neuropsychologische Biomarker sind.

5. Verfahren nach einem der Ansprüche 1 bis 4, wobei das untersuchte Subjekt ein Tier ist, vorzugsweise ein Säugetier, bevorzugter ein Mensch.

6. Verfahren nach einem der Ansprüche 1 bis 5, wobei bei dem Schritt eines Bereitstellens des numerischen Modells (NM) die stochastische Approximation in einem Erwartungs-Maximierungsverfahren eine stochastische Approximation einer ErwartungsMaximierungstechnik einer Monte-Carlo-Markov-Chain ist.

7. Verfahren nach einem der Ansprüche 1 bis 6, wobei bei dem Schritt eines Bereitstellens des numerischen Modells (NM) die Anzahl der Subjekten größer oder gleich 100 ist.

8. Verfahren nach einem der Ansprüche 1 bis 7, wobei in dem numerischen Modell (NM) die Streuung der Progressionskurve für das biologische Phänomen unter einer Vielzahl von Subjekten als Standardabweichungen der Zeit für eine Vielzahl von Werten von Biomarkern bereitgestellt werden.

9. Verfahren zum Vorhersagen, dass ein Subjekt ein Risiko hat, an einer mit Adipositas zusammenhängenden Krankheit zu leiden, das Verfahren zum Vorhersagen mindestens umfassend die folgenden Schritte:

   - Durchführen der Schritte des Verfahrens zum Bestimmen des zeitlichen Verlaufs eines biologischen Phänomens, das ein untersuchtes Subjekt nach einem der Ansprüche 1 bis 8 betreffen kann, wobei das biologische Phänomen die Krankheit ist, um einen ersten zeitlichen Verlauf zu erlangen, und
   - Vorhersagen, dass das Subjekt ein Risiko hat, an einer mit Adipositas zusammenhängenden Krankheit zu leiden, basierend auf den Fettcharakterisierungsparametern.

10. Verfahren zum Diagnostizieren einer Krankheit, das Verfahren zum Diagnostizieren mindestens umfassend den folgenden Schritt:

   - Durchführen der Schritte des Verfahrens zum Bestimmen des zeitlichen Verlaufs eines biologischen Phänomens, das ein untersuchtes Subjekt nach einem der Ansprüche 1 bis 8 betreffen kann, wobei das biologische Phänomen die Krankheit ist, um einen ersten zeitlichen Verlauf zu erlangen, und
   - Diagnostizieren der Krankheit basierend auf dem ersten zeitlichen Verlauf.

11. Verfahren zum Identifizieren eines Biomarkers, wobei der Biomarker ein diagnostischer Biomarker einer Pathologie, ein Anfälligkeits-Biomarker einer Pathologie, ein prognostischer Biomarker einer Pathologie oder ein prädiktiver Biomarker als Reaktion auf die Behandlung einer Pathologie ist, das Verfahren umfassend die folgenden Schritte:

- Durchführen des Verfahrens zum Bestimmen des zeitlichen Verlaufs eines biologischen Phänomens, das ein untersuchtes Subjekt betreffen kann, nach einem der Ansprüche 1 bis 8, wobei die ersten Daten Daten in Bezug auf ein Subjekt sind, das an der Pathologie leidet, um einen ersten zeitlichen Verlauf zu erlangen,
- Durchführen des Verfahrens zum Bestimmen des zeitlichen Verlaufs eines biologischen Phänomens, das ein untersuchtes Subjekt betreffen kann, nach einem der Ansprüche 1 bis 8, wobei die ersten Daten Daten in Bezug auf ein Subjekt sind, das nicht an der Pathologie leidet, um einen zweiten zeitlichen Verlauf zu erlangen,
- Auswählen eines Biomarkers basierend auf dem Vergleich des ersten und des zweiten zeitlichen Verlaufs.

12. Computerprogrammprodukt, umfassend Anweisungen zum Durchführen der Schritte eines Verfahrens nach einem der Ansprüche 1 bis 11, wenn das Computerprogrammprodukt auf einer geeigneten Computervorrichtung ausgeführt wird.

13. Computerlesbares Medium, auf dem ein Computerprogrammprodukt nach Anspruch 12 kodiert ist.

**Revendications**

1. Procédé mis en œuvre par ordinateur pour déterminer la progression temporelle d'un phénomène biologique qui peut affecter un sujet étudié, le phénomène biologique étant une maladie ou le vieillissement, le procédé comprenant les étapes de :

- fourniture de premières données, les premières données étant des biomarqueurs de la progression de la maladie ou du vieillissement pour le sujet étudié,
- fourniture d'un modèle numérique (NM), le modèle numérique (NM) étant une fonction dans une conduite de Riemann (RM), le modèle numérique (NM) associant à des valeurs des biomarqueurs une trajectoire de progression temporelle du phénomène biologique et la dispersion de la trajectoire de progression du phénomène biologique parmi une pluralité de sujets, le modèle numérique (NM) étant obtenu en utilisant une approximation stochastique dans une technique de maximisation de l'espérance sur des biomarqueurs prélevés à différents points dans le temps pour une pluralité de sujets,
- conversion des premières données en au moins un point sur la même conduite de Riemann (RM), et
- utilisation du modèle numérique (MN) sur l'au moins un point obtenu à l'étape de conversion pour déterminer une progression temporelle du phénomène biologique pour le sujet étudié.

2. Procédé selon la revendication 1, dans lequel le phénomène biologique est un phénomène biologique dont la progression temporelle s'étend sur plus de trois ans.

3. Procédé selon la revendication 1 ou 2, dans lequel le phénomène biologique est une maladie neurodégénérative.

4. Procédé selon l'une quelconque des revendications 1 à 3, dans lequel à l'étape de fourniture de premières données, les premières données sont des biomarqueurs neuropsychologiques.

5. Procédé selon l'une des revendications 1 à 4, dans lequel le sujet étudié est un animal, de préférence un mammifère, plus préférentiellement un être humain.

6. Procédé selon l'une quelconque des revendications 1 à 5, dans lequel, à l'étape de fourniture du modèle numérique (NM), l'approximation stochastique dans une technique de maximisation de l'espérance est une technique de maximisation de l'espérance par approximation stochastique de Monte-Carlo par chaînes de Markov.

7. Procédé selon l'une quelconque des revendications 1 à 6, dans lequel, à l'étape de fourniture du modèle numérique (MN), le nombre de sujets est supérieur ou égal à 100.

8. Procédé selon l'une quelconque des revendications 1 à 7, dans lequel, dans le modèle numérique (NM), la dispersion de la trajectoire de progression du phénomène biologique parmi une pluralité de sujets est fournie sous forme d'écarts types du temps pour une pluralité de valeurs de biomarqueurs.

9. Procédé permettant de prédire qu'un sujet présente un risque de souffrir d'une maladie, le procédé de prédiction comprenant au moins l'étape de :

   - mise en œuvre des étapes du procédé de détermination de la progression temporelle d'un phénomène biologique qui peut affecter un sujet étudié selon l'une quelconque des revendications 1 à 8, le phénomène biologique étant la maladie, pour obtenir une première progression temporelle, et
   - prédiction que le sujet présente un risque de souffrir de la maladie sur la base de la première progression temporelle.

10. Procédé de diagnostic d'une maladie, le procédé de diagnostic comprenant au moins l'étape de :

    - mise en œuvre des étapes du procédé de détermination de la progression temporelle d'un phénomène biologique qui peut affecter un sujet étudié selon l'une quelconque des revendications 1 à 8, le phénomène biologique étant la maladie, pour obtenir une première progression temporelle, et
    - diagnostic de la maladie sur la base de la première progression temporelle.

11. Procédé d'identification d'un biomarqueur, le biomarqueur étant un biomarqueur diagnostique d'une pathologie, un biomarqueur de sensibilité d'une pathologie, un biomarqueur pronostique d'une pathologie ou un biomarqueur prédictif en réponse au traitement d'une pathologie, le procédé comprenant les étapes de :

    - mise en œuvre du procédé de détermination de la progression temporelle d'un phénomène biologique qui peut affecter un sujet étudié selon l'une quelconque des revendications 1 à 8, les premières données étant des données relatives à un sujet atteint de la pathologie, pour obtenir une première progression temporelle,
    - mise en œuvre du procédé de détermination de la progression temporelle d'un phénomène biologique qui peut affecter un sujet étudié selon l'une quelconque des revendications 1 à 8, les premières données étant des données relatives à un sujet non atteint de la pathologie, pour obtenir une seconde progression temporelle,
    - sélection d'un biomarqueur sur la base de la comparaison des première et seconde progressions temporelles.

12. Produit de programme informatique comprenant des instructions pour mettre en œuvre les étapes d'un procédé selon l'une quelconque des revendications 1 à 11 lorsque ledit produit de programme informatique est exécuté sur un dispositif informatique approprié.

13. Support lisible par ordinateur sur lequel est codé un produit de programme informatique selon la revendication 12.

FIG.1

FIG.2

<u>FIG.3</u>

<u>FIG.4</u>

$$FIG.5$$

$$FIG.6$$

FIG.7

Satiotemporal analysis of longitudinal manifold-valued data

FIG.8

FIG.9

FIG.10

FIG.11

FIG.12

FIG.13

FIG.14

FIG.15

FIG.16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004242972 A1

**Non-patent literature cited in the description**

- A multivariate nonlinear mixed effects model for longitudinal image analysis : Application to amyloid imaging. **BILGEL MURAT et al.** NEUROIMAGE. ELSEVIER, 18 April 2016, vol. 134, 658-670

- **SCHIRATTI**. Mixed-effects model for the spatiotemporal analysis of longitudinal manifold-valued data. *MATHEMATICAL FOUNDATIONS OF COMPUTATIONAL ANATOMY*, 09 October 2015, 48-59